# EUROPEAN PATENT APPLICATION

(11) **EP 4 768 600 A1**
(43) Date of publication of application: **01.07.2026**
(21) Application number: 24859652.0
(22) Date of filing: 23.08.2024
(51) Int. Cl.: C12Q 1/6869, A61K 31/519, A61K 45/00, A61P 11/00, A61P 35/00, A61P 43/00, C12Q 1/6886, G01N 33/53, G01N 33/574

(54) **METHOD FOR ACQUIRING DATA ON EFFICACY OF CDK4/6 INHIBITOR IN LUNG CANCER**

(30) Priority: 25.08.2023 JP 2023137612
(71) Applicant: Keio University, Tokyo, 108-8345 (JP)
(72) Inventor: YASUDA, Hiroyuki, Tokyo 160-8582 (JP); SATO, Toshiro, Tokyo 160-8582 (JP); SHINOZAKI, Taro, Tokyo 160-8582 (JP); HAMAMOTO, Junko, Tokyo 160-8582 (JP); FUKUNAGA, Koichi, Tokyo 160-8582 (JP)
(74) Representative: Cabinet Becker et Associés
(86) International application number: PCT/JP2024/030034
(87) International publication number: WO 2025/047619

(57) **Abstract**

Provided is a method for acquiring data on efficacy of a CDK4/6 inhibitor in a lung cancer cell, the method including detecting EGFR gene mutation, and CDKN2A/B gene deletion or loss-of-function mutation in a lung cancer cell derived from a lung cancer patient. Presence of EGFR gene mutation, and CDKN2A/B gene deletion or loss-of-function mutation indicates that the CDK4/6 inhibitor has efficacy.

## Description

### Technical Field

The present invention relates to a method for predicting efficacy of a CDK4/6 inhibitor in treatment of subjects afflicted with lung cancer.

### Background Art

It is said that, globally, two million or more people get lung cancer in a year. According to epidemiological data, among them, about 500000 people are assumed to have EGFR (Epidermal Growth Factor Receptor) gene mutation-positive lung adenocarcinoma.

EGFR gene mutation-positive lung adenocarcinoma is one of the largest subgroups of lung adenocarcinoma. For EGFR gene mutation-positive lung adenocarcinoma, EGFR tyrosine kinase inhibitors as molecularly targeted therapeutic drugs have been developed, and the efficacy thereof was confirmed in clinical trials. Among them, osimertinib (trade name TAGRISSO) which is a third-generation EGFR tyrosine kinase inhibitor is widely used globally from the viewpoint of efficacy and safety. The EGFR tyrosine kinase inhibitors exhibit efficacy (objective response rate) of about 70% with respect to EGFR gene mutation-positive lung adenocarcinoma, and can control disease conditions at the early stage of the treatment.

Meanwhile, for a certain number of patients, the EGFR tyrosine kinase inhibitor cannot be used at all due to side effects, complication, and the like. Furthermore, not a few lung cancers have been confirmed to have treatment resistance (primary resistance) from the initial phase after the start of treatment with the EGFR tyrosine kinase inhibitor, and not a few lung cancers acquire resistance (acquired resistance) when about one to two years have passed after the start of the treatment. Therefore, development of a treatment strategy is expected for lung cancers of patients for whom the EGFR tyrosine kinase inhibitor cannot be used, and lung cancers which have primary resistance or acquired resistance.

A CDK4/6 inhibitor is a selective inhibitor for cyclin-dependent kinase CDK4 and CDK6, and palbociclib and abemaciclib are low molecular weight compounds used as therapeutic drugs for hormone receptor (HR) positive and HER2-negative breast cancers.

Non-Patent Literature 1 indicates that moderate antitumor activity is exhibited by administering palbociclib alone to a patient with non-small cell lung cancer having CDKN2A/B gene deletion or loss-of-function mutation.

Non-Patent Literature 2 indicates that 82% of patients who have EGFR gene mutation were cyclin D1-positive, and administering abemaciclib alone as a CDK4/6 inhibitor was not effective for both a cell line having EGFR gene mutation and a cell line having no EGFR mutant gene in a growth inhibition test of a cell line derived from non-small cell lung cancer having EGFR gene mutation, but a concentration of a drug necessary for 50% growth inhibition was reduced when osimertinib and abemaciclib were used in combination.

### Citation List

### Non-Patent Literature

NPTL 1: Eugene R. Ahn, et al., Precision Oncology no. 4 (2020) 757-766. Published online June 25, 2020.
NPTL 2: Osoegawa et. al., Investigational New Drugs 41, 183-192 (2023) 2023 Feb 15. doi: 10.1007/s10637-023-01337-8

### Summary of Invention

### Technical Problem

Non-Patent Literature 1 does not include description as to presence or absence of EGFR gene mutation in the patients. Non-Patent Literature 2 indicates that cyclin D1 is expressed in many of the patients having EGFR gene mutation, but does not describe connection with CDKN2A/B gene deletion or loss-of-function mutation, and neither indicates nor suggests that accuracy for predicting efficacy of a CDK4/6 inhibitor is enhanced by combining CDKN2A/B gene deletion or loss-of-function mutation with EGFR gene mutation.

To date, a biomarker which accurately predicts a therapeutic effect of a CDK4/6 inhibitor in lung cancer has not been identified.

An object to be achieved by the present invention is to provide a method for acquiring data on efficacy of a CDK4/6 inhibitor in lung cancer.

### Solution to Problem

The present invention includes embodiments described below.

### Item 1.

A method for acquiring data on efficacy of a CDK4/6 inhibitor in a lung cancer cell, the method including detecting EGFR gene mutation, and CDKN2A/B gene deletion or loss-of-function mutation in a lung cancer cell derived from a lung cancer patient.

### Item 2.

The method according to item 1, wherein, when EGFR gene mutation, and CDKN2A/B gene deletion or loss-of-function mutation are present, the presence indicates that the CDK4/6 inhibitor has efficacy.

### Item 3.

A method for acquiring data on efficacy of a CDK4/6 inhibitor in a lung cancer cell having EGFR gene mutation, the method including detecting CDKN2A/B gene deletion or loss-of-function mutation in the lung cancer cell having EGFR gene mutation.

### Item 4.

The method according to item 3, wherein, when CDKN2A/B gene deletion or loss-of-function mutation is present, the presence indicates that the CDK4/6 inhibitor has efficacy.

### Item 5.

A method for acquiring data on efficacy of a CDK4/6 inhibitor in a lung cancer cell having CDKN2A/B gene deletion or loss-of-function mutation, the method including detecting EGFR gene mutation.

### Item 6.

The method according to item 5, wherein, when EGFR gene mutation is present, the presence indicates that the CDK4/6 inhibitor has efficacy.

### Item 7.

The method according to any one of items 1 to 6, wherein the CDK4/6 inhibitor includes palbociclib or abemaciclib.

### Item 8.

A kit for acquiring data on efficacy of a CDK4/6 inhibitor in a lung cancer cell, the kit including a reagent for measuring EGFR gene mutation and a reagent for measuring CDKN2A/B gene deletion or loss-of-function mutation.

### Item 9.

A therapeutic agent for lung cancer having EGFR gene mutation, and CDKN2A/B gene deletion or loss-of-function mutation, the therapeutic agent including a CDK4/6 inhibitor as an active ingredient.

### Brief Description of Drawings

Fig. 1 shows an outline of a mouse xenograft experiment.
Fig. 2 shows photographs of various immunohistochemical staining of organoids and xenograft tumors, in which the scale bar: 100 µm.
Fig. 3 shows outlines of genotype-phenotype relationships in an EGFR-TKI resistance mechanism and organoid genetic abnormality in whole exome sequencing analysis, in which n=31. LUAD; Lung Adenocarcinoma, SQ; Squamous Cell Carcinoma, SCLC; Small Cell Carcinoma, LCNEC; large cell neuroendocrine carcinoma. The horizontal axis represents names of organoid lines.
Fig. 4A shows a result of a cell viability assay in which a plurality of palbociclib concentrations are used, in which CDKN2A/B loss: EGFR gene variant in which CDKN2A/B gene deletion is present (n=8), CDKN2A/B WT: EGFR gene variant in which a CDKN2A/B gene is wild-type (n=5).
Fig. 4B shows IC₅₀ values for CDKN2A/B gene-deleted lines (n=8) and wild-type ones (n=5), in which ** represents p<0.01, t test. Error bar: standard deviation.
Fig. 5 shows average tumor volumes (mm³) of xenograft tumors treated with palbociclib (n=5) or a vehicle (n=5) for CDKN2A/B gene-deleted lines ((A) E-12, (B) E-14, (C) E-17), and (D) CDKN2A/B wild-type line (E-01B), in which *** represents p<0.001, ** represents p<0.01, t test. N.S. no significant difference.
Fig. 6A shows a result of a cell viability assay in which a plurality of abemaciclib concentrations are used, for EGFR gene variant in which CDKN2A/B gene deletion is present (n=3) and EGFR gene variant in which a CDKN2A/B gene is wild-type (n=3), in which CDKN2A/B loss: EGFR gene variant in which CDKN2A/B gene deletion is present, CDKN2A/B WT: EGFR gene variant in which a CDKN2A/B gene is wild-type, error bar: standard deviation.
Fig. 6B shows a result of a cell viability assay in which a plurality of palbociclib concentrations are used, for KRAS gene variant in which CDKN2A/B gene deletion is present (n=3) and KRAS gene variant in which CDKN2A/B is wild-type (n=1), in which CDKN2A/B loss: KRAS gene variant in which CDKN2A/B gene deletion is present, CDKN2A/B WT: KRAS gene variant in which CDKN2A/B gene is wild-type, error bar: standard deviation.

### Description of Embodiments

In this description, "comprise" conceptually includes "consist essentially of" and "consist of".

In one aspect of the present invention, a method for acquiring data on efficacy of a CDK4/6 inhibitor in a lung cancer cell is provided, and the method includes detecting EGFR gene mutation, and CDKN2A/B gene deletion or loss-of-function mutation in a lung cancer cell derived from a lung cancer patient.

In a case where EGFR gene mutation, and CDKN2A/B gene deletion or loss-of-function mutation are present, the presence indicates that the CDK4/6 inhibitor has efficacy for treatment of the lung cancer of the subject.

The timing when the EGFR gene mutation is detected, and the timing when the CDKN2A/B gene deletion or loss-of-function mutation is detected may be different from each other or the same. For example, the timing when the EGFR gene mutation is detected may be earlier or later than the timing when the CDKN2A/B gene deletion or loss-of-function mutation is detected.

In another aspect of the present invention, a method for acquiring data on efficacy of a CDK4/6 inhibitor in a lung cancer cell having EGFR gene mutation is provided, and the method includes detecting CDKN2A/B gene deletion or loss-of-function mutation in the lung cancer cell having EGFR gene mutation.

The lung cancer cell having the EGFR gene mutation is preferably a lung cancer cell which has EGFR gene mutation and is derived from a lung cancer patient or collected from a lung cancer patient.

In a case where CDKN2A/B gene deletion or loss-of-function mutation is present, the presence indicates that the CDK4/6 inhibitor has efficacy for the lung cancer of the subject.

In still another aspect of the present invention, a method for acquiring data on efficacy of a CDK4/6 inhibitor in a lung cancer cell having CDKN2A/B gene deletion or loss-of-function mutation is provided, and the method includes detecting EGFR gene mutation in the lung cancer cell.

In a case where EGFR gene mutation is present, the presence indicates that the CDK4/6 inhibitor has efficacy.

The EGFR gene mutation refers to structural abnormality of a gene of an EGFR (epidermal growth factor receptor) that is a HER family member. The genome sequence of a human EGFR is represented by NCBI Gene ID 1956 and the amino acid sequence thereof is represented by UNIProt P00533. The genome sequence of a mouse EGFR is represented by NCBI Gene ID: 24329 and the amino acid sequence thereof is represented by UNIProt Q01279.

Examples of the EGFR gene mutation include gene mutation in an exon 18-21 region. Examples of the deletion mutation include amino acid deletion, and further include amino acid deletion involving amino acid substitution. Particularly, mutation in which one amino acid or a plurality of continuous amino acids in a codon E746-I759 region of exon 18 are deleted often occurs, and examples of the mutation include simple deletion of five amino acids in codons E746-A750, insertion of S in L747-E753 deletion, simple deletion of L747-E751, insertion of P in L747-E750 deletion, point mutation in codon 719 of exon 18 (G719X, collectively represented by X since amino acid may be A, C, S), point mutation in codon 709 of exon 18 (E709X, G719X, collectively represented by X since amino acid may be K, A), deletion mutation in exon 19, insertion mutation in exon 20, point mutation of S768I of exon 20, point mutation of L858R of exon 21, and point mutation of L861Q of exon 21. EGFR gene mutation is registered in the COSMIC (the catalogue of somatic mutations in cancer, https://cancer.sanger.ac.uk/cosmic) database. These EGFR gene mutations are publicly known, and can be detected by using a well-known or a publicly known detection method or by using a commercially available kit.

Among these EGFR gene mutations, mutation in which a phosphorylation signal is continuously sent to a growth signaling pathway on the downstream side even when stimulation of a ligand such as EGF and TGFα does not functionally occur is called activating mutation.

In one embodiment, EGFR gene mutation refers to the following EGFR gene mutation which clearly has clinical significance in relation to lung cancer according to "Guide to EGFR gene mutation test for lung cancer patients, the 5.0-th edition (Haigan kanja niokeru EGFR idenshi hen'i kensa no tebiki dai 5.0 han" (December 16, 2021), of The Japan Lung Cancer Society. (1) EGFR gene mutation which is already known as activating mutation.
·Exon 19 deletion mutation, L858R mutation
·G719X mutation, L861Q mutation, S768I mutation
·Exon 20 insertion mutation (A763_Y764insFQEA)

CDKN2A/B gene deletion or loss-of-function mutation refers to gene deletion or loss-of-function mutation of at least one gene of CDKN2A (cyclin-dependent kinase inhibitor 2A) and CDKN2B (cyclin-dependent kinase inhibitor 2B). The genome sequence of human CDKN2A is represented by NCBI Gene ID: 1029, the amino acid sequence thereof is represented by UNIProt P42771, the genome sequence of human CDKN2B is represented by NCBI Gene ID: 1030, the amino acid sequence thereof is represented by UNIProt Q5ZEY8, the genome sequence of mouse CDKN2A is represented by NCBI Gene ID: 12578, the amino acid sequence thereof is represented by UNIProt Q64364, the genome sequence of mouse CDKN2B is represented by NCBI Gene ID: 12579, and the amino acid sequence thereof is represented by UNIProt Q549R4.

CDKN2Aand CDKN2B are each a tumor suppressor gene having connection with control of a cell cycle, and deletion or loss-of-function mutation of a CDKN2A gene and a CDKN2B gene has connection with abnormality in cell cycle control and progress of cancer. Deletion or loss-of-function mutation of a CDKN2A gene and a CDKN2B gene can be detected by using a publicly known detection method or by using a commercially available kit.

CDKN2A/B gene deletion or loss-of-function mutation is not particularly limited as long as the CDKN2A/B gene deletion or loss-of-function mutation is such mutation that the function of the gene is lost. The CDKN2A gene and the CDKN2B gene are adjacent to each other in the chromosomal region 9p21, and a large region including both the CDKN2A gene and the CDKN2B gene is often deleted. The CDKN2A/B genes are preferably deleted at both of two alleles.

The CDKN2A/B gene deletion includes 9p21 homozygous deletion. In the 9p21 homozygous deletion, the same region may not necessarily be deleted at both alleles, and deletion may be deletion in which the functions of the CDKN2A gene and the CDKN2B gene are lost at each allele.

The loss-of-function mutation of the CDKN2A/B gene includes missense mutation and nonsense mutation. The missense mutation is, for example, D84X (H, N, Y) (means that D at position 84 in the amino acid sequence is mutated to H, N, or Y) or D108X (H, N, Y). The nonsense mutation is, for example, W110*. The loss-of-function mutation of the CDKN2A/B gene includes CDKN2A/B gene silencing due to methylation. For example, silencing of the CDKN2A/B gene occurs due to methylation of a promoter of the CDKN2A/B gene.

CDKN2A/B gene deletion or loss-of-function mutation includes a state in which deletion, missense mutation, or nonsense mutation occurs at one of the two alleles, and methylation occurs at the other of the two alleles.

The inventors of the present invention have established a patient-derived EGFR gene mutation-positive lung cancer organoid library of 31 lines, and have examined connection between genetic abnormality and efficacy of a CDK4/6 inhibitor. In the examination, the inventors have unexpectedly found that, in a case where CDKN2A/B gene deletion or loss-of-function mutation is present in addition to EGFR gene mutation being positive, efficacy of the CDK4/6 inhibitor can be predicted at high specificity. Combination of the two factors of "EGFR gene mutation" and "CDKN2A/B gene deletion or loss-of-function mutation" is important as a biomarker that can enhance accuracy for predicting efficacy of a CDK4/6 inhibitor in a lung cancer patient.

Examples of the lung cancer include at least one selected from the group consisting of lung adenocarcinoma, squamous cell carcinoma, small cell carcinoma, large cell carcinoma, mixed type carcinoma thereof, and lung cancer which cannot be classified into any histological type.

Examples of the CDK4/6 inhibitor include, but are not limited to, palbociclib, abemaciclib, and ribociclib. The CDK4/6 inhibitor preferably includes palbociclib or abemaciclib.

Presence or absence of EGFR gene mutation, and CDKN2A/B gene deletion or loss-of-function mutation is an index for predicting a treatment effect of the CDK4/6 inhibitor for a subject afflicted with lung cancer, or for predicting efficacy of the CDK4/6 inhibitor in treatment of a subject afflicted with lung cancer.

**In** a lung cancer cell collected from a subject, EGFR gene mutation, and CDKN2A/B gene deletion or loss-of-function mutation are detected. In a case where both EGFR gene mutation, and CDKN2A/B gene deletion or loss-of-function mutation are present, the CDK4/6 inhibitor is evaluated as being highly likely to be effective for treatment of lung cancer of the subject.

A test of EGFR gene mutation for lung cancer tissues has already been conducted clinically. Furthermore, as a test in deletion or loss-of-function mutation for a CDKN2A/B gene, p16 immunostaining is conducted in an otolaryngologic tumor region or the like, and, furthermore, oncogene panel tests such as "OncoGuide NCC Oncopanel", "FoundationOne CDx cancer genome profile", and "FoundationOne Liquid CDx cancer genome profile", and a deletion test in a 9p21 region by a fluorescence in situ hybridization (FISH) method, can also be used. A group for which the CDK4/6 inhibitor is effective can be selected by combination of these tests which have already been conducted in clinical sites.

Thus, by using the method according to the embodiment of the present invention, lung cancer patients for whom the CDK4/6 inhibitor exhibits efficacy can be simply selected with high accuracy, and, furthermore, a treatment method suitable for the patient can be selected.

In a case where, in the above-described detection step, a subject in which both EGFR gene mutation, and CDKN2A/B gene deletion or loss-of-function mutation have been detected in a lung cancer cell, that is, data indicating that both EGFR gene mutation, and CDKN2A/B gene deletion or loss-of-function mutation are present, and the CDK4/6 inhibitor is effective for treatment of lung cancer of the subject, is obtained, administration of the CDK4/6 inhibitor can be further performed. Thus, a subject in which both EGFR gene mutation, and CDKN2A/B gene deletion or loss-of-function mutation have been detected in a lung cancer cell can be treated.

In one aspect of the present invention, a kit for acquiring data on efficacy of a CDK4/6 inhibitor in treatment of a subject afflicted with lung cancer is provided, and the kit includes a reagent for measuring EGFR gene mutation, and a reagent for measuring CDKN2A/B gene deletion or loss-of-function mutation.

In one embodiment, the reagent for measuring EGFR gene mutation is a probe for detecting mutation of an EGFR gene. In another embodiment, the reagent for measuring EGFR gene mutation is a probe for amplifying or detecting an EGFR gene. The probe is preferably a nucleic acid probe, and more preferably RNA or DNA.

In one embodiment, the reagent for measuring CDKN2A/B gene deletion or loss-of-function mutation is a probe for detecting CDKN2A/B gene deletion or loss-of-function mutation.

In another embodiment, the reagent for measuring CDKN2A/B gene deletion or loss-of-function mutation is a probe for amplifying or detecting a CDKN2A/B gene. The probe is preferably a nucleic acid probe, and more preferably RNA or DNA.

A method in which RNA or cDNA is obtained from a lung cancer cell collected from a sample, and target nucleic acid is detected by using a nucleic acid probe by a well-known method such as a nucleic acid amplification method or nucleic acid hybridization method, is well known.

In another embodiment, the reagent for measuring CDKN2A/B gene deletion or loss-of-function mutation is an antibody to an expression product of a CDKN2A/B gene. For example, presence or absence of CDKN2A/B gene deletion or loss-of-function mutation in a lung cancer cell collected from a subject can be detected by using an antibody specific to protein that is a wild-type or a null mutant expression product of a CDKN2A/B gene. For example, in a case where a measured value of an expression product of a CDKN2A/B gene in a cell collected from a subject is less as compared with a level of an expression product of a CDKN2A/B gene in a normal lung cell, the measured value represents data indicating that CDKN2A/B gene deletion or loss-of-function mutation is present.

The kit may further include an instruction manual, for the kit, which is used when data on efficacy of a CDK4/6 inhibitor in a lung cancer cell is acquired.

The kit may further include a control sample. The control sample represents, for example, an expression level of a gene in a normal lung cell. In one embodiment, in a case where an expression level of a CDKN2A/B gene in a lung cancer cell collected from a subject is lower as compared with an expression level of a CDKN2A/B gene in a normal lung cell, CDKN2A/B gene deletion or loss-of-function mutation may be present.

In one aspect of the present invention, a therapeutic agent for lung cancer having EGFR gene mutation, and CDKN2A/B gene deletion or loss-of-function mutation is provided, and the therapeutic agent includes the CDK4/6 inhibitor as an active ingredient.

In one embodiment, the therapeutic agent is a therapeutic agent for lung cancer in a subject selected since, in detection of EGFR gene mutation, and CDKN2A/B gene deletion or loss-of-function mutation in a lung cancer cell collected from a subject, both EGFR gene mutation, and CDKN2A/B gene deletion or loss-of-function mutation are present. In such a subject, the CDK4/6 inhibitor is highly likely to be effective for treatment of the lung cancer of the subject.

An amount of the CDK4/6 inhibitor to be administered per one day depends on an age, a body weight, a symptom, and the like of a patient, but may be about 0.1 to 5000 mg per one day for an adult (body weight of 50 kg) in general, and is preferably 1 to 1000 mg and more preferably 10 to 1000 mg. This amount of the CDK4/6 inhibitor is preferably administered once a day or dividedly administered about two to three times a day. The administration may be parenteral administration such as intravenous injection, intraperitoneal injection, subcutaneous injection, or intramuscular injection, or oral administration. The inhibitor to be administered may contain a pharmacologically acceptable carrier, diluent, or excipient. The form of the inhibitor is not limited, and, for example, the inhibitor may be orally administered by a tablet, a capsule, a granule, powder, syrup, or the like, or may be parenterally administered by injection, drops, suppository, spray, or the like.

A dosing schedule is selected as appropriate according to conditions such as a kind of the CDK4/6 inhibitor, presence or absence of a concomitant drug, a prior treatment history, a disease stage, presence or absence of metastasis, and patient's age and sex.

There are a large number of EGFR gene mutation-positive lung cancer patients around the world, and the number of patients with lung cancer having EGFR gene mutation, and CDKN2A/B gene deletion or loss-of-function mutation is also considered to be substantially large. Therefore, it is considered that the treatment in which the method according to the present invention is used can be performed in countries around the world. **In** a case where such a patient can be treated by a different kind of drug from an EGFR tyrosine kinase inhibitor, the treatment can be expected to lead to enhancement of treatment outcomes.

The present disclosure further includes embodiments described below.

### Item 1.

A method for acquiring data on efficacy of a CDK4/6 inhibitor in a lung cancer cell, the method including
detecting EGFR gene mutation, and CDKN2A/B gene deletion or loss-of-function mutation in a lung cancer cell derived from a lung cancer patient.

### Item 2.

The method according to Item 1, wherein, when EGFR gene mutation, and CDKN2A/B gene deletion or loss-of-function mutation are present, the presence indicates that the CDK4/6 inhibitor has efficacy.

### Item 3.

A method for acquiring data on efficacy of a CDK4/6 inhibitor in a lung cancer cell having EGFR gene mutation, the method including
detecting CDKN2A/B gene deletion or loss-of-function mutation in the lung cancer cell having EGFR gene mutation.

### Item 4.

The method according to Item 3, wherein, when CDKN2A/B gene deletion or loss-of-function mutation is present, the presence indicates that the CDK4/6 inhibitor has efficacy.

### Item 5.

A method for acquiring data on efficacy of a CDK4/6 inhibitor in a lung cancer cell having CDKN2A/B gene deletion or loss-of-function mutation, the method including
detecting EGFR gene mutation.

### Item 6.

The method according to Item 5, wherein, when EGFR gene mutation is present, the presence indicates that the CDK4/6 inhibitor has efficacy.

### Item 7.

The method according to any one of Items 1 to 6, wherein the CDK4/6 inhibitor includes palbociclib or abemaciclib.

### Item 8.

A kit for acquiring data on efficacy of a CDK4/6 inhibitor in a lung cancer cell, the kit including a reagent for measuring EGFR gene mutation and a reagent for measuring CDKN2A/B gene deletion or loss-of-function mutation.

### Item 9.

A therapeutic agent for lung cancer having EGFR gene mutation, and CDKN2A/B gene deletion or loss-of-function mutation, the therapeutic agent including a CDK4/6 inhibitor as an active ingredient.

### Item 10.

The method according to Item 3, wherein the lung cancer cell having EGFR gene mutation includes a lung cancer cell having EGFR gene mutation derived from a lung cancer patient.

### Item 11.

A method for predicting efficacy of a CDK4/6 inhibitor in treatment of a subject afflicted with lung cancer, the method including
detecting EGFR gene mutation, and CDKN2A/B gene deletion or loss-of-function mutation in a lung cancer cell collected from the subject, and
making an evaluation that the CDK4/6 inhibitor is highly likely to be effective for treatment of the lung cancer of the subject when EGFR gene mutation, and CDKN2A/B gene deletion or loss-of-function mutation are present.

### Item 12.

A method for predicting efficacy of a CDK4/6 inhibitor in treatment of a subject afflicted with lung cancer, a lung cancer cell of the subject having EGFR gene mutation, the method including
detecting CDKN2A/B gene deletion or loss-of-function mutation in the lung cancer cell collected from the subject, and
making an evaluation that the CDK4/6 inhibitor is highly likely to be effective for treatment of the lung cancer of the subject when CDKN2A/B gene deletion or loss-of-function mutation is present.

### Item 13.

A method for predicting efficacy of a CDK4/6 inhibitor in treatment of a subject afflicted with lung cancer, a lung cancer cell of the subject having CDKN2A/B gene deletion or loss-of-function mutation, the method including
detecting EGFR gene mutation in the lung cancer cell collected from the subject, and
making an evaluation that the CDK4/6 inhibitor is highly likely to be effective for treatment of the lung cancer of the subject when EGFR gene mutation is present.

### Item 14.

The method according to any one of Items 11 to 13, wherein the CDK4/6 inhibitor includes palbociclib or abemaciclib.

### Item 15.

A kit for predicting efficacy of a CDK4/6 inhibitor in treatment of a subject afflicted with lung cancer by a method including the following steps (1) and (2), the kit including a reagent for measuring EGFR gene mutation, and a reagent for measuring CDKN2A/B gene deletion or loss-of-function mutation,
(1) detecting EGFR gene mutation, and CDKN2A/B gene deletion or loss-of-function mutation in a lung cancer cell collected from the subject, and
(2) making an evaluation that the CDK4/6 inhibitor is highly likely to be effective for treatment of the lung cancer of the subject when EGFR gene mutation, and CDKN2A/B gene deletion or loss-of-function mutation are present.

### Item 16.

A therapeutic agent for lung cancer having EGFR gene mutation, and CDKN2A/B gene deletion or loss-of-function mutation, the therapeutic agent including a CDK4/6 inhibitor as an active ingredient.

### Examples

### 1. Method

### Establishment of EGFR gene mutation-positive lung cancer organoid

This study was approved by the Institutional Review Board of Keio University Hospital, Kawasaki Municipal Hospital, and Saiseikai Central Hospital. Written informed consent was obtained from all of patients involved in this study. Clinical specimens of patients with lung cancer having EGFR gene mutation for which the EGFR gene mutation was identified by a clinically known method were sequentially collected from May, 2018 to April, 2022. The clinical specimens used in this study included surgical resection, bronchoscopic biopsy, CT-guided needle biopsy, ascites, pleural effusion, and autopsy specimens. The lung cancer organoid was established by the method previously reported by us (Cell Rep. 2023 Mar 28;42(3):112212). This will be briefly described. For a tissue, a sample was finely cut into 1 mm³ fragments by using surgical scissors. The fragments were digested by Liberase TH (Roche) at 37°C for 30 minutes. The digested pellets were treated with TrypLE Express (Thermo Fisher Scientific) at 37°C for 10 minutes. The isolated cell was embedded in Matrigel Growth Factor Reduced (Coming) droplets, to laminate a medium. The organoid from the pleural effusion was established in accordance with the above-described report (Cell Rep. 2023 Mar 28;42(3):112212).

### Immunohistochemical staining

Immunohistochemical staining was performed in accordance with the above-described report (Cell Rep. 2023 Mar 28;42(3):112212). Briefly, patient specimens and isolated xenograft were fixed with 4% paraformaldehyde. The tissue block of the organoid was prepared by using iPgell (GSPG20-1, Nippon Genetics Co., Ltd.) in accordance with a manual of the manufacturer. Standard protocols were used for sectioning of paraffin embedded tissues and hematoxylin-eosin (H&E) staining. Antibodies used for the immunohistochemical staining included rabbit anti-synaptophysin (proteintec, 17785-1-AP, 1:1500), mouse anti-CD56 (DAKO, M7304, 1:200), rabbit anti-chromogranin A (abcam, ab15160, 1:1000), mouse anti-CDKN2A/p16INK4a (Gene Tex, GTX01783, 1:100), mouse anti-TTF1 (DAKO, M3575, 1:100), mouse anti-p63 (DAKO, M7317, 1:100), mouse anti-p40 (Nichirei, bc28, 1:2), and rabbit anti-AQP5 (abcam, ab92320, 1:20).

### Whole exome sequencing (WES) analysis

The WES analysis was performed in accordance with the above-described report (Cell Rep. 2023 Mar 28;42(3):112212). This will be briefly described. For DNA extraction, a QIAamp Blood Mini Kit (QIAGEN) was used. The quality of DNA was checked by agarose gel electrophoresis. A 150 bp paired-end library was prepared by using a SureSelect Human All Exon V6 kit (Agilent) in accordance with the protocol of the manufacturer. The sequencing was performed by an Illumina Novaseq device. By using BWA-MEM version 0.7.17 (http://bio-bwa.sourceforge.net/) [Li, H. & Durbin, R. Fast and accurate short read alignment with Burrows-Wheeler transform. Bioinformatics 25, 1754-1760 (2009)], mapping of a trimmed fastq file to human reference genome version GRCh37 (hg19) was performed. Somatic mutation, single nucleotide mutation (SNV), insertion, and deletion were detected by using Genome Analysis Toolkit version 4.1.9.0 (https://gatk.broadinstitute.org). A variant was detected by using Mutect2. In order to eliminate germline variants, the detected variants were filtered by eliminating variants which were detected at allele frequency of 0.1% or more by dbSNP or a Japanese germline variant database (Human Gene Mutation Database). Furthermore, variants were filtered by eliminating ones detected in at least two samples among 35 blood samples and 23 normal organoids of which the sequences were determined in the previous study (Cell Rep. 2023 Mar 28;42(3):112212) of the inventors of the present invention. Data was deposited with a database of DNA Data Bank of Japan (DDBJ) under Accession Nos. JGAS000610 (study) and JGAD000739 (dataset).

### In vitro cell viability assay

The cell viability assay was performed by using CellTiter-Glo (manufactured by Promega) in accordance with the protocol of the manufacturer as reported by the inventors of the present invention (Cell Rep. 2023 Mar 28;42(3):112212). Each organoid established in "Establishment of EGFR gene mutation-positive lung cancer organoid" was seeded into a 96-well plate (1500 cells/well), and treated with palbociclib and abemaciclib. A control cell was treated with DMSO as a vehicle having the same concentration. An absorbance was processed by Cytation5 (BioTek, #MO1210), and the measurement was made on the seventh day after that. The IC50 value was calculated by Prism software (GraphPad Prism 7.04). With 1 µM of palbociclib or abemaciclib, cell viability(/DMSO)≥75% was determined as resistant, and cell viability<75% was determined as sensitive.

### Organoid xenograft and animal experiment

All of the animal experiments were approved by the Laboratory Animal Center, Keio University School of Medicine. The xenograft experiment was performed in accordance with the above-described report (Cell Rep. 2023 Mar 28;42(3):112212). Female NOG (NOD/Shi-scid, IL-2RγKO Jic) mice (5 weeks old, about 20 g/mouse) were purchased from Charles River Laboratories Japan, Inc. For each transplant, an organoid cluster (about 1x10⁵ cells) was suspended in 50 µL of Matrigel, and was subcutaneously transplanted into the mice. The mice were euthanized two or three months after the transplant.

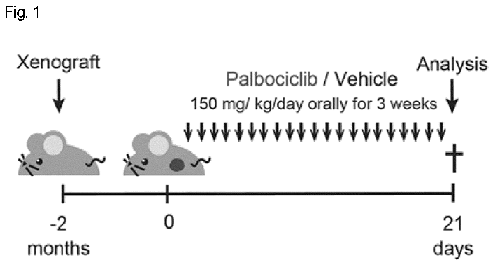

In an in vivo efficacy experiment of palbociclib as a CDK4/6 inhibitor, the organoid cluster was transplanted, and administration of the drug was started at a time when the tumor volume (TV) of the xenograft became 100 mm³ or more (Fig. 1). The tumor volume (TV) was calculated as TV=(L×W×H/2) by measuring the length (L), the width (W), and the height (H) of each tumor with use of a caliper. The mice were randomly allocated to a vehicle administration group (n=5) or palbociclib (150 mg/kg/day) administration group (n=5). Oral administration of the drug was continuously performed daily for three weeks, and the TVs and the body weights were measured at a frequency of 2 to 3 times per week. After the three weeks of drug administration, the mice were sacrificed and tumors were isolated.

### Statistical analysis

The values of the IC50, the tumor volume, or immunohistochemistry were determined by using a Wilcoxon rank sum test or a chi-squared test. The significance level was represented by a P value in each experiment. In the figure, the asterisks represent the following: * represents P value<0.05, ** represents P value<0.01, *** represents P value<0.001, and n.s. represents P value>0.05. The statistical analysis was performed by using GraphPad Prism version 7.04 statistical software (GraphPad Prism Corp., Califomia, USA).

### 2. Results

### Establishment of lung cancer organoid library

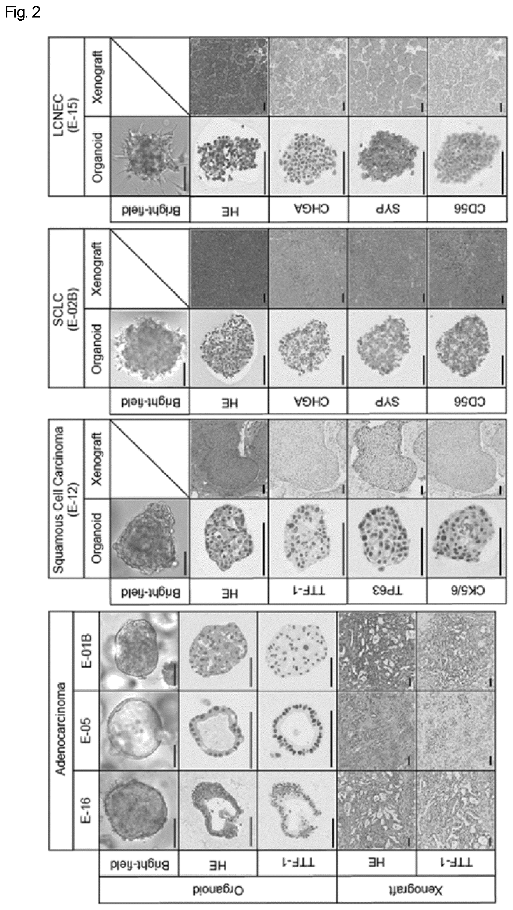

The inventors of the present invention established 31 lineages of lung cancer organoids by using various clinical specimens obtained through surgical resection, bronchoscopic biopsy, CT-guided needle biopsy, ascites, pleural effusion, or autopsy. Furthermore, immunohistochemical staining was performed for the established organoids and the xenograft tumors (lung adenocarcinoma (E-16, E-05, E-01B lines), squamous cell lung carcinoma (E-12 line), small cell lung cancer (E-02B line), and large cell neuroendocrine carcinoma (E-15), each line was EGFR gene mutation-positive), and the organoids were confirmed to be experimental models reflecting actual clinical practice (Fig. 2).

### Genotype and phenotype

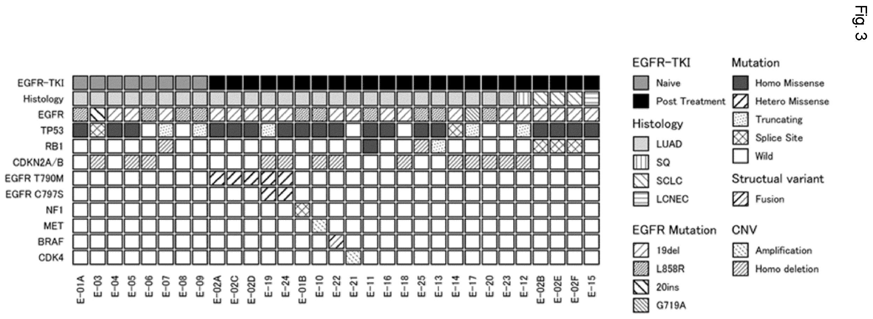

Whole exome sequencing (WES) was performed for the established 31-lineage organoids, and various genetic abnormalities having connection with EGFR gene mutation and resistance were confirmed (Fig. 3).

### Treatment effect of CDK4/6 inhibitor for lung cancer having EGFR gene mutation in which CDKN2A/B gene deletion or loss-of-function mutation was present

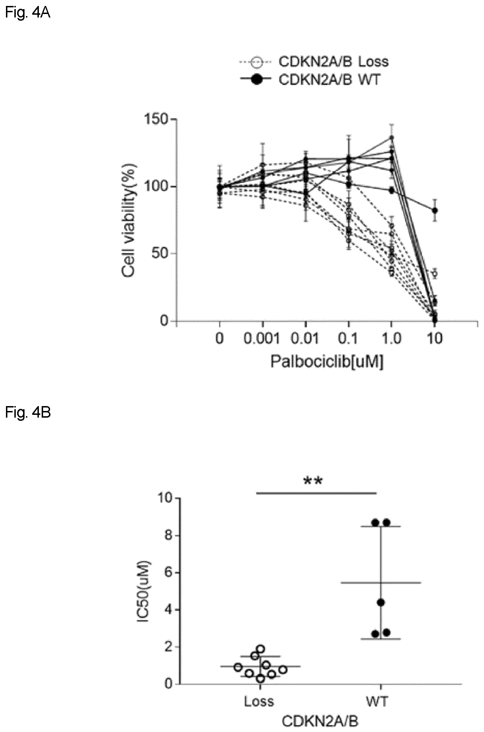

According to the in vitro cell viability assay in Fig. 4A, in the lung cancer organoid line of each of an EGFR gene mutation-positive line (lines of Homo deletion in the CDKN2A/B row in Fig. 3) in which CDKN2A/B gene deletion was present, and an EGFR gene mutation-positive line in which CDKN2A/B was wild-type, the cell viability was reduced by palbociclib having a high concentration of 10 µM. However, when the concentration of palbociclib was 1 µM, the cell viability became less than 75% by the palbociclib in all the examples of the EGFR gene mutation-positive lines in which CDKN2A/B gene deletion was present, and the cell viability was more than 75% in all the examples of the EGFR gene mutation-positive lines in which CDKN2A/B gene was wild-type.

With reference to Fig. 4B, the IC₅₀ value was significantly low in the EGFR gene mutation-positive line in which CDKN2A/B gene deletion was present as compared with the EGFR gene mutation-positive line in which CDKN2A/B was wild-type, and it is indicated that the treatment effect of palbociclib was high.

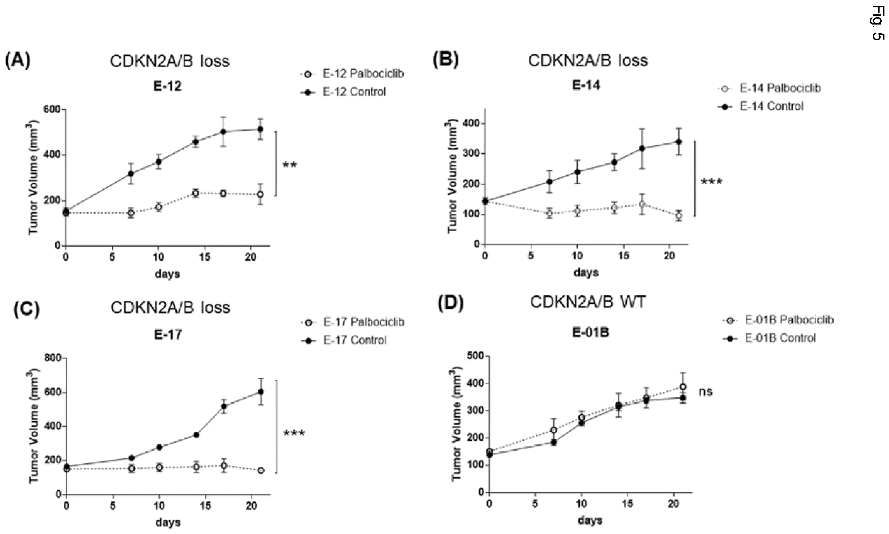

With reference to Fig. 5, in the squamous cell carcinoma-derived line E-12 having EGFR gene mutation (exon 19 deletion (19del)) and CDKN2A/B gene deletion, the lung adenocarcinoma-derived line E-14 having EGFR gene mutation (19del) and CDKN2A/B gene deletion, and the lung adenocarcinoma-derived line E-17 having EGFR gene mutation (G719A) and CDKN2A/B gene deletion, the average tumor volume of the xenograft tumor on the 21st day was significantly reduced in the palbociclib administration group as compared with the vehicle administration group ((A) to (C)). Meanwhile, the average tumor volume of the xenograft tumor in the line (E-01B) having EGFR gene mutation (L858R) and a wild-type CDKN2A/B gene was not significantly different between the palbociclib administration group and the vehicle administration group (D).

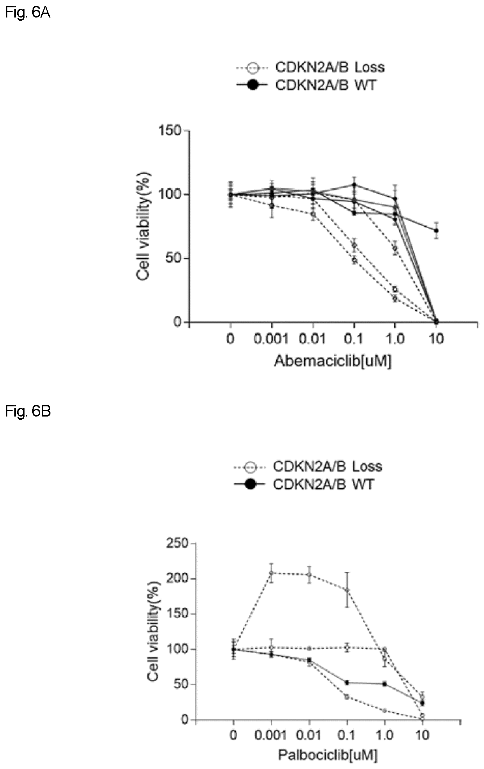

With reference to Fig. 6A, the EGFR gene variant in which CDKN2A/B gene deletion was present was abemaciclib sensitive in all the examples, and the EGFR gene variant in which the CDKN2A/B gene was wild-type was abemaciclib resistant in all the examples.

With reference to Fig. 6B, a KRAS gene variant in which CDKN2A/B gene deletion or loss-of-function mutation was present, was found to be unuseful as a biomarker since palbociclib sensitive ones and palbociclib resistant ones coexisted. The KRAS gene is located on the downstream side of EGFR signaling, and gene mutation thereof promotes constitutive cell proliferation similarly to mutant EGFR. Even combination of CDKN2A/B gene deletion or loss-of-function mutation, and KRAS gene mutation was not useful as a CDK4/6 inhibitor sensitive biomarker.

Table 1 indicates connection between presence or absence of mutation of each gene (EGFR gene, KRAS gene, CDKN2A/B gene), and sensitivity/resistance with respect to palbociclib, in 25 lung cancer organoid lines. The 25 lines included ones overlapping the organoid lines used in the above-described experiment. In a case where a "CDKN2A/B gene deletion or loss-of-function mutation-positive" and "EGFR gene mutation-positive" biomarker was regarded as a useful biomarker, all of 10 lines of positive biomarkers were palbociclib sensitive, and 6 lines were palbociclib sensitive and 9 lines were palbociclib resistant in 15 lines of negative biomarkers. The specificity was 100%.

**[Table 1]**

| Biomarker | | Palbociclib sensitive | Palbociclib resistant | Specificity |
|---|---|---|---|---|
| Having CDKN2A/B deletion or loss-of-function mutation | Positive | 13 | 2 | 78% |
| | Negative | 3 | 7 | |
| Having both CDKN2A/B deletion or loss-of-function mutation, and EGFR mutation | Positive | 10 | 0 | 100% |
| | Negative | 6 | 9 | |
| Having both CDKN2A/B deletion or loss-of-function mutation, and KRAS mutation | Positive | 2 | 2 | 78% |
| | Negative | 14 | 7 | |

Four organoid lines of squamous cell lung carcinoma were established, and connection with palbociclib sensitivity/resistance was examined. In a case where a "CDKN2A/B gene deletion or loss-of-function mutation-positive" and "EGFR gene mutation-positive" biomarker was regarded as a useful biomarker, 1 line of a positive biomarker was palbociclib sensitive, and 1 line was palbociclib resistant and 2 lines were palbociclib sensitive in 3 lines of negative biomarkers. The specificity was 100%.

The data provides preclinical evidence that supports use of a CDK4/6 inhibitor for treatment of lung cancer having "CDKN2A/B gene deletion or loss-of-function mutation", and "EGFR gene mutation". The result obtained by the inventors of the present invention suggests that a CDK inhibitor, in particular, a CDK4/6 inhibitor is likely to be effective for treatment of lung cancer having "CDKN2A/B gene deletion or loss-of-function mutation", and "EGFR gene mutation".

## Claims

1. A method for acquiring data on efficacy of a CDK4/6 inhibitor in a lung cancer cell, the method comprising
detecting EGFR gene mutation, and CDKN2A/B gene deletion or loss-of-function mutation in a lung cancer cell derived from a lung cancer patient.

2. The method according to claim 1, wherein, when EGFR gene mutation, and CDKN2A/B gene deletion or loss-of-function mutation are present, the presence indicates that the CDK4/6 inhibitor has efficacy.

3. A method for acquiring data on efficacy of a CDK4/6 inhibitor in a lung cancer cell having EGFR gene mutation, the method comprising
detecting CDKN2A/B gene deletion or loss-of-function mutation in the lung cancer cell having EGFR gene mutation.

4. The method according to claim 3, wherein, when CDKN2A/B gene deletion or loss-of-function mutation is present, the presence indicates that the CDK4/6 inhibitor has efficacy.

5. A method for acquiring data on efficacy of a CDK4/6 inhibitor in a lung cancer cell having CDKN2A/B gene deletion or loss-of-function mutation, the method comprising
detecting EGFR gene mutation.

6. The method according to claim 5, wherein, when EGFR gene mutation is present, the presence indicates that the CDK4/6 inhibitor has efficacy.

7. The method according to any one of claims 1 to 6, wherein the CDK4/6 inhibitor includes palbociclib or abemaciclib.

8. A kit for acquiring data on efficacy of a CDK4/6 inhibitor in a lung cancer cell, the kit comprising a reagent for measuring EGFR gene mutation and a reagent for measuring CDKN2A/B gene deletion or loss-of-function mutation.

9. A therapeutic agent for lung cancer having EGFR gene mutation, and CDKN2A/B gene deletion or loss-of-function mutation, the therapeutic agent comprising a CDK4/6 inhibitor as an active ingredient.
